# EUROPEAN PATENT SPECIFICATION

(11) **EP 2 756 068 B1**
(45) Date of publication and mention of the grant of the patent: **25.11.2015**
(21) Application number: 12759432.3
(22) Date of filing: 14.09.2012
(51) Int. Cl.: C12M 1/12, C12N 1/12

(54) **PROCESS FOR THE PREPARATION OF BIOFUEL USING ALGAE**
VERFAHREN ZUR HERSTELLUNG EINES BIOTREIBSTOFFS AUS ALGEN
PROCÉDÉ DE PRÉPARATION DE BIOCARBURANT AU MOYEN D'ALGUES

(30) Priority: 15.09.2011 EP 11007525
(43) Date of publication of application: 23.07.2014
(73) Proprietor: Energy Knowledge Group BV, 6416 SG Heerlen (NL)
(72) Inventor: KOVÁCS, Sándor, NL-6191 GH Beek (NL); BRANDTS, Wim, NL-6336 SX Hulsberg (NL)
(74) Representative: Renkema, Jaap
(86) International application number: PCT/EP2012/068063
(87) International publication number: WO 2013/037938

(56) References cited:
- WO-A1-98/28083
- US-A1- 2002 079 270
- US-A1- 2007 243 607
- HENDERSON RITA K ET AL: "Surfactants as bubble surface modifiers in the flotation of algae: Dissolved air flotation that utilizes a chemically modified bubble surface", ENVIRONMENTAL SCIENCE & TECHNOLOGY, vol. 42, no. 13, July 2008 (2008-07), pages 4883-4888, XP002665572, ISSN: 0013-936X, DOI: 10.1021/ES702649H

## Description

The invention relates to a process for the preparation of algae oil and to a process for the preparation of biofuel.

The emission of carbon dioxide (CO₂) from the burning of fossil fuels has been identified as the major contributor to global warming and climate change. Flue- (or off-) gases that are produced in industrial processes contain CO₂. Flue gases from processes for producing electricity and especially those that produce electricity from fossil fuels, such as coals (brown coals and black coals) are rich in CO₂.

However, for the immediate term over the next 20 - 30 years at least, the world will continue to rely on fossil fuels as the primary source of energy. The challenge for the fossil fuel industry is to find cost-effective solutions that will reduce the emission of carbon dioxide into the atmosphere, since in many industrialized nations industrial plants, such as power plants are subject to, or will become subject into economic penalties for the emission of various substances, particularly carbon dioxide, to the environment. For example, controls on carbon dioxide emissions result in emissions trading and taxes, and require permits. As the cost of emission penalties increases, there is a lot of effort to capture CO₂ from flue gases and to either store it or convert the CO₂ into something useful.

A very elegant solution is the conversion of carbon dioxide from industrial off-gases/flue gases into biofuel. This has a dual advantage: CO₂ emission is reduced, while at the same time the need for fossil fuels as the primary source of energy is also reduced.

One way to convert carbon dioxide into biofuel, is by making use of algae that like plants, use daylight for the process of photosynthesis. Algae can convert the energy of the daylight into algae oil using CO₂. This algae oil may be further processed into biofuel. Industrial algae cultivation will absorb CO₂ from the atmosphere or, in more concentrated form, from other CO₂ sources (e.g. CO₂ captured from industrial flue-gases from power plants, refineries, etc.). Burning algae oil releases only what it absorbed in the first place, resulting in a balanced "carbon neutral" effect. This makes algae oil the environmentally-friendly oil.

In the right environment, fresh algae cells grow and divide exponentially, doubling every few hours, while absorbing available nutrients, CO₂ and light energy. Instead of waiting hundreds of millions of years for algae to become a fossil fuel, industrial processes can transform algae into oil in a matter of days.

There are two main methods of cultivation of algae for the production of algae oil; algae may be cultivated in open ponds or in photobioreactors, which are closed systems.

The open systems, in order to increase their efficiency, are generally designed as a continuous culture in which a fixed supply of culture medium or influent ensures constant dilution of the system. The organisms adapt their growth rate to this dilution regime, with the organism best adapted to the environment prevailing in the system winning the competition with the other organisms.

A drawback of the common open algae culture systems is the major risk of contamination by undesirable photosynthetic micro-organisms which can be introduced via air or rain. Also, open algae culture systems are typically operated in a batch mode and since reaction conditions are not easy to control, it is difficult to estimate when the algae oil produced is ready to be harvested.

To reduce the possibility of parasite infestation, photobioreactors, which are closed systems, are preferred. In these closed systems, the process conditions, such as evaporation of growth medium, gas transfer and temperature, can be accurately controlled so that a higher productivity may be achieved. Furthermore, photobioreactors have a large surface-to-volume ratio, thereby offering a good efficiency in using light, which also improves the productivity (conversion to algae oil). Also, typically, the culture density of algae produced is 10 to 20 times greater than bag culture in which the culturing of algae is done in bags - and can be even greater.

However, a problem with photobioreactors is that due to the growth of the algae, fouling of the inside of the photobioreactor occurs. Fouling is not desired, since it decreases the amount of light with which the algae can do photosynthesis and thus decreases the production rate. Furthermore, to remove fouling of the light-transmitting surfaces of reactors, photobioreactors must be frequently shut down for their mechanical cleaning and sterilization. During this shut down, valuable production time is wasted.

In order to reduce the amount of production time lost, tube self-cleaning mechanisms have been developed. However, even with these self-cleaning mechanisms, fouling still occurs (although less fast) and the photobioreactors still need to be cleaned.

Therefore, it is an object of the invention to provide a process for the production of algae oil from algae in a photobioreactor wherein fouling of the photobioreactor is drastically reduced and may even be non-existent.

This object is achieved by a process for the production of algae oil comprising the steps of
a) mixing algae, nutrients, CO₂, a surfactant and an aqueous medium comprising water to form an aqueous dispersion of algae-containing bubbles comprising algae, nutrients, CO₂, and water and
b) exposing the dispersion to light to produce algae oil-containing bubbles comprising algae oil.

This process is a very clean process and hardly any fouling of the wall of the photobioreactor occurs, even after longer cultivation times. Since fouling hardly occurs, the process can be run for a very long time without needing intermediate cleaning. Furthermore, since there is hardly any fouling, light can reach the algae with a high intensity and the process can be run with a high efficiency.

As an additional advantage, the aqueous dispersion comprising the algae-containing bubbles can contain higher concentrations CO₂ than what is maximally possible in water (under normal conditions, the amount of CO₂ that can be present in water has a maximum of around 5wt% based on water). This makes the process for the production of algae oil more efficient in terms of yield of amount of algae oil that may be produced per liter water. In other words, a smaller volume may be used to get the same amount of algae oil.

As an additional advantage, it has been found that the algae-containing bubbles may concentrate the light, thereby increasing the speed of production of algae oil by the algae.

Another additional advantage may be that the algae-containing bubbles are very stable, which makes them very suitable for transport through e.g. tubes when using a carrier liquid, such as an aqueous medium comprising water and which makes them especially suitable for transport through a tubular photobioreactor.

High rates of mass transfer may thus be achieved without damaging the cultured algae cells and/or suppression of their growth. This as opposed to the known systems for algae growth, where shear forces occuring during the transport of the algae, damage the cultured algae cells and/or suppress their growth.

Furthermore, as the algae-containing bubbles by themselves are a closed environment, they are also very suitable for use in open algae culture systems, as 1) infections are less likely (since the algae-containing bubbles are closed) and 2) conditions can be controlled better as the algae-containing bubbles in essence protect the algae culture from external factors (or mitigate the effect of external conditions) and the starting conditions inside the bubbles are fixed in the preparation of the bubbles.

Furthermore, the algae-containing bubbles may contain high concentrations of CO₂ and this would make the production of algae-oil more efficient.

In principle any algae that can produce algae oil from CO₂ may be used. Typically, the algae used for algae oil production are single-celled microalgae, with cells that are usually less than 0.4mm in diameter. However, research is being done into using seaweeds for algae oils, probably due to the high availability of this resource. Preferably, in the process of the invention, use is made of single-celled microalgae. Single-celled microalgae are commonly found in ponds, lakes and marine environments.

Examples of algae oil producing single-celled microalgae are known to the person skilled in the art and include but are not limited to the following algae: *Botryococcus braunii, Chlorella, Parachlorella, Dunaliella tertiolecta, Dunaliella saline, Gracilaria, Pleurochrysis carterae (also called CCMP647), Ulva* and *Sargassum.*

Also, the following microalgae from the microalgae culture collection from the US Department of Energy's Aquatic Species Program, may be able to produce algae oil: *Bacillariophyceae,* for example *Bacillariophyceae Achnantes orientalis, Bacillariophyceae amphora coffeiformis, Bacillariophyceae amphora delicatissima capitata, Bacillariophyceae amphora coffeiformis punctata, Bacillariophyceae amphora delicatissima, Bacillariophyceae amphora coffeiformis linea, Bacillariophyceae amphora sp., Bacillariophyceae amphora coffeiformis tenuis, Bacillariophyceae amphora coffeiformis taylori, Bacillariophyceae chaetoceros muelleri, Bacillariophyceae chaetoceros gracilis, Bacillariophyceae chaetoceros sp., Bacillariophyceae chaetoceros muelleri subsalsum, Bacillariophyceae chaetoceros muelleri-trans, Bacillariophyceae chaetoceros muelleri muelleri, Bacillariophyceae chaetoceros sp., Bacillariophyceae chroomonas sp, Bacillariophyceae chrysosphaera sp., Bacillariophyceae sarcinoid chrysophyte, Bacillariophyceae cricosphaera sp, Bacillariophyceae cryptomonas sp. Bacillariophyceae cyclotella cryptica, Bacillariophyceae cyclotella cryptica, Bacillariophyceae cyclotella meneghiniana, Bacillariophyceae cyclotella sp. Bacillariophyceae navicula sp. nov, Bacillariophyceae dunaliella sp, Bacillariophyceae amphiprora hyalina, Bacillariophyceae navicula saprophila, Bacillariophyceae navicula pseudotenelloides, Bacillariophyceae navicula biskanterae, Bacillariophyceae navicula acceptata, Bacillariophyceae navicula, Bacillariophyceae nitzschia pusilla monoensis, Bacillariophyceae nitzschia pusilla elliptica, Bacillariophyceae nitzschia alexandrina, Bacillariophyceae nitzschia quadrangula, Bacillariophyceae nitzschia inconspicua, Bacillariophyceae nitzschia microcephala, Bacillariophyceae nitzschia pusilla, Bacillariophyceae nitzschia dissipata, Bacillariophyceae nitzschia communis, Bacillariophyceae nitzschia hantzschiana, Bacillariophyceae nitzschia frustulum, Bacillariophyceae nitzschia sp., Bacillariophyceae phaeodactylum tricornatum, Bacillariophyceae phaeodactylum tricornutum, Bacillariophyceae thalassiosira weissflogii; Chlorophyceae, for example Chlorophyceae ankistrodesmus falcatus, Chlorophyceae borodinella sp. Chlorophyceae botryococcus braunii, Chlorophyceae chlorococcum sp., Chlorophyceae chlorella sp., Chlorophyceae chlorella ellipsoidea, Chlorophyceae chlorella salina, Chlorophyceae franceia sp, Chlorophyceae monoraphidium sp., Chlorophyceae monoraphidium minutum, Chlorophyceae nannochloris sp., Chlorophyceae oocystis pusilla, Chlorophyceae oocystis pusilla, Chlorophyceae oocystis parva, Chlorophyceae oocystis sp., Chlorophyceae oscillatoria limnetica, Chlorophyceae oscillatoria subbrevis, Chlorophyceae oscillatoria sp., Chlorophyceae platymonas sp., Chlorophyceae stichococcus sp; Cyanophyceae, for example Cyanophyceae Gleocapsa sp., Cyanophyceae synechococcus sp.; Chlamydophyceae, for example Chlamydophyceae chlorococcales; Eustigmatophyceae, for example Eustigmatophyceae ellipsoidon sp., Eustigmatophyceae eustigmatophyte, Eustigmatophyceae nannochloropsis salina; Prymnesiophyceae, for example Prymnesiophyceae hymenomonas sp, Prymnesiophyceae isochrysis aff. glabana, Prymnesiophyceae pleurochrysis dentata, Prymnesiophyceae pleurochrysis sp.; Chrysophyceae, for example Chrysophyceae ochromonas sp.; Chrysophyceae pavlova sp.; Cyanophyceae, for example Cyanophyceae pseudoanabaena sp.; Prasinophyceae,* for example *Prasinophyceae pyramimonas sp., Prasinophyceae tetraselmis suecica, Prasinophyceae tetraselmis sp.;*

Preferably, in the process of the invention, use is made of algae that are capable of taking up high amounts of CO₂ as in the algae-containing bubbles produced by the process of the invention, the CO₂ concentration can be much higher than in water. In case the microalgae is capable of taking up high amounts of CO₂, the process can be much more efficient and the yield of algae oil may be much higher.

Preferably, in the process of the invention use is made of a single-celled microalgae, preferably of a *Parachlorella* species. For example the algae may be *Parachlorella kessleri, Chlorella vulgaris, Botryococcus braunii, Emilyania Huxleyi Coccolith,* preferably *Parachlorella kessleri, Chlorella vulgaris, Emilyania Huxleyi coccolith, Dunaliella tertiolecta or Dunaliella saline* more preferably *Parachlorella kessleri, Chlorella vulgaris, Dunaliella tertiolecta* or *Dunaliella saline,* most preferably *Parachlorella kessleri.*

The algae used in the process of the invention may be a monoculture, but may also be a mixture of algae cultures, for example a mixture of any of the algae as described herein.

The algae used may be algae of any color, for example the algae may be green algae, red algae, golden-brown algae or brown algae.

The algae which are mixed with other components to obtain the algae-containing bubbles are preferably taken from an algae stockculture. From this stockculture, small amounts may be taken for mixing with the other components to obtain the algae-containing bubbles. During the process of the invention, the algae stockculture can be continuously run, so that the small amounts of algae taken for use in the process of the invention can be replaced by freshly cultured algae.

In general, the optimal reaction conditions for the algae in the algae-containing bubbles depend on the algae strain chosen. For example, the temperature of cultivation for marine algae may be in the range from 16 to 27°C, for example from 18-24°C. The salinity of the aqueous medium comprising water used for mixing with the algae may be in the range from 12 to 40g/l, for example in the range form 20-24g/l and the pH may be in the range from 7-9, for example from 8.2-8.7 and the light intensity may be in the range of 1,000-10,000 lux, for example 2,500-5,000 lux (depending on the density of the algae culture). The photoperiod, that is the amount of hours that the algae are exposed to light and the amount of hours that the algae are not exposed to light in a period of 24 hours, is preferably from 16 hours of light and 8 hours of dark to 24 hours of light to 0 hours of dark. For example, the optimal reaction conditions for *Parachlorella kessleri* are a temperature in the range of 34 to 36°C and/or a pH can be in the range of 5 to 7 (in case the acidification of CO₂ in the aqueous medium comprising water is not compensated for) or in the range of 7 to 9 (as larger amounts of algae-oil may then be obtained) and/or the salinity of the aqueous medium comprising water is essentially 0 (fresh water and/or the photoperiod is preferably from 16 hours of light and 8 hours of dark to 24 hours of light to 0 hours of dark.

The mixing of the algae, nutrients, CO₂, surfactant and the aqueous medium comprising water to form a dispersion of algae-containing bubbles may be performed by any method known in the art to be suitable for producing bubbles, for example the algae-containing bubbles may be formed using mechanical mixers, in-line share mixers, etc.

Preferably, in the process of the invention the algae, nutrients, CO₂, surfactant and an aqueous medium comprising water are mixed using a submersible ultrasonic transducer (14; 140; 240). Therefore, the invention also relates to a process according to the invention, wherein step a) is performed by operating a submersible ultrasonic transducer (14; 140; 240), preferably at a frequency in the range from 20 to 25kHz, to form the algae-containing bubbles (15; 150; 250). Using a submersible ultrasonic transducer has the advantage that the process is easily upscaleable to produce large amounts of dispersion of algae-containing bubbles. Furthermore, it is easier to control homogeneity of the algae-containing bubbles formed and also a cost-effective solution for the production of the algae-containing bubbles.

The optimal frequency at which the submersible ultrasonic transducer is preferably operated for an optimal production of algae oil by the algae in the algae-containing bubbles can be determined through routine experimentation by the person skilled in the art and may depend for example on the type of algae, nutrients, surfactant and amount of CO₂, surfactant and an aqueous medium comprising water used. Preferably, in the process of the invention, the frequency of the submersible ultrasonic transducer is at least 10kHz, for example at least 15kHz, preferably at least 20kHz and /or at most 35kHz, for example at most 30kHz, preferably at most 25kHz.

Preferably, the submersible ultrasonic transducer is operated at a frequency in the range from 20 to 25kHz to produce the algae-containing bubbles. At this frequency, the algae-containing bubbles produced will be more homogeneous and formation of the dispersion of algae-containing bubbles is most efficient.

As used herein, with submersible ultrasonic transducers are meant ultrasonic transducers that can be completely submerged in a liquid and that can be operated while being completely submerged in a liquid. This in contrast to conventional ultrasonic transducers (also referred to herein as ultrasonic cleaners), which cannot be operated while being completely submerged in a liquid, but instead are operated by placing them on the outside of a containing comprising a liquid to transduce the ultrasonic waves into the liquid.

Submersible ultrasonic transducers are commercially available from for example Sonic systems inc., Zenith Mfg. & Chemical Corp, Hielscher Ultrasonics GmbH., etc.

The shape of the submersible ultrasonic transducer is in principle not critical; for example the submersible ultrasonic transducer may have the shape of a rod or of a cuboid, for example a rectangular cuboid.

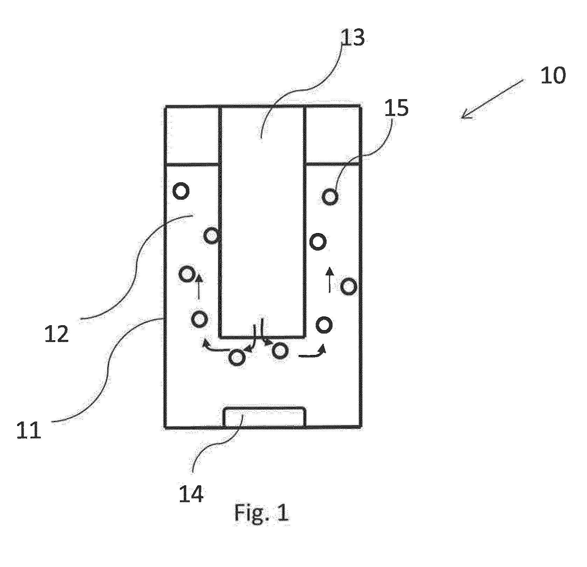

The mixing of algae, nutrients, CO₂, surfactant and an aqueous medium comprising water may be performed in one mixing unit, for example mixing unit 10 as represented by Fig. 1.

Fig. 1 schematically illustrates a mixing unit 10 comprising a container 11 containing a first liquid 12, a porous element 13 which is at least partially submerged in the first liquid and a submersible ultrasonic transducer 14 which is at least partially, but preferably completely submerged in the first liquid and wherein the first liquid is an aqueous medium comprising water.

Therefore, the invention also relates to a process according to the invention, wherein step a) is performed using a mixing unit (10) comprising
- a container (11) containing a first liquid (12)
- a porous element (13) which is at least partially submerged in the first liquid and
- a submersible ultrasonic transducer (14) which is at least partially submerged in the first liquid and wherein the first liquid is an aqueous medium comprising water,
wherein step a) involves
- leading a second liquid and/or a first gas stream into the container, preferably through the porous element, wherein the second liquid is an aqueous medium comprising water and CO₂, wherein the first gas stream is a gas comprising CO₂
- adding the nutrients, algae and the surfactant to the container, preferably through the porous element and
- operating the submersible ultrasonic transducer at a frequency in the range from 20 to 25kHz to produce the aqueous dispersion of algae-containing bubbles (15).

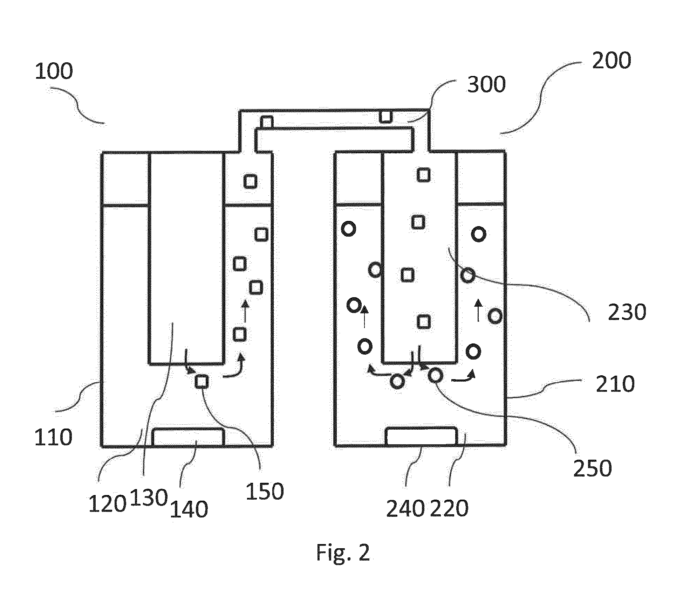

Alternatively and preferably, the mixing of algae, nutrients, CO₂, surfactant and an aqueous medium comprising water may be performed in two mixing units, for example mixing units 100 and 200 as represented by Fig. 2.

Fig. 2. schematically illustrates a mixing unit 100 comprising a container 110 containing a third liquid 120, a porous element 130 which is at least partially submerged in the third liquid and a first submersible ultrasonic transducer 140 which is at least partially, but preferably completely submerged in the third liquid and wherein the third liquid is an aqueous medium comprising water and a second mixing unit 200 comprising a container 210 containing a fourth liquid 220 and optionally a porous element 230 which is at least partially submerged in the fourth liquid and a second submersible ultrasonic transducer 240 which is at least partially, but preferably completely submerged in the fourth liquid and wherein the fourth liquid is an aqueous medium comprising water and a transporting means 300 connecting the third liquid to the fourth liquid.

Therefore, the invention preferably relates to a process according to the invention, wherein step a) is performed using a mixing unit (100) comprising
- a first container (110) containing a third liquid (120),
- a porous element (130) which is at least partially submerged in the third liquid and
- a first submersible ultrasonic transducer (140) which is at least partially submerged in the third liquid and
wherein the third liquid is an aqueous medium comprising water and
a second mixing unit (200) comprising
- a second container (210) containing a fourth liquid (220),
- a porous element (230) which is at least partially submerged in the fourth liquid and
- a second submersible ultrasonic transducer (240) which is at least partially submerged in the fourth liquid and wherein the fourth liquid is an aqueous medium comprising water and a transporting means (300) connecting the third liquid to the fourth liquid wherein step a) involves
- leading a second liquid and/or a second gas stream into the second container, preferably through the porous element, wherein the second liquid is an aqueous medium comprising water and CO₂, wherein the second gas stream is a gas comprising CO₂
- adding algae to the second container, preferably through the porous element
- operating the first submersible ultrasonic transducer at a frequency in the range from 20 to 25kHz to produce an aqueous dispersion of bubbles (150) comprising nutrients, CO₂ and water and
- subsequently leading the aqueous dispersion of bubbles comprising nutrients, CO₂, and water into the second container, preferably through the porous element using the transporting means and
- operating the second submersible transducer at a frequency in the range from 20 to 25kHz to produce an aqueous dispersion of algae-containing bubbles comprising algae, CO₂, nutrients and water (250).

In the process of the invention, the algae-containing bubbles comprising the algae, nutrients, CO₂ and water are formed with a high efficiency (when using the method with two mixing units as described above, up to 80-85% of the material added is formed into the dispersion of algae-containing bubbles and the remaining material may be recycled for another mixing step).

Furthermore, algae-containing bubbles formed in the process of the invention are very stable. The aqueous dispersion of bubbles comprising nutrients, CO₂ and water may be led into the fourth liquid through the optional porous element (230). The aqueous dispersion of bubbles comprising nutrients, CO₂ and water - upon formation of the dispersion- may spontaneously flow from the container 110 into the transporting means to flow into the fourth liquid, in which case no additional energy (from for example a pump) is needed to lead the bubbles comprising nutrients, CO₂ and water into the fourth liquid.

Preferably, all components to be added for the preparation of the algae-containing bubbles (the algae, the nutrients, CO₂, the surfactant, the aqueous medium comprising water) are added through the porous element(s) (13, 130 and/or 230).

The order of addition of the algae, the nutrients, CO₂, the surfactant, the aqueous medium comprising water to the container (11, 110 and/or 210) is in principle not critical.

In one embodiment, CO₂ is added first, after which the other components may be added.

It is clear to the skilled person that the algae, nutrients, CO₂, a surfactant and an aqueous medium comprising water may be added as separate components, but may also be added in pre-mixes thereof, for example the aqueous medium comprising water may further comprise the algae and/or CO₂. For example the surfactant and the nutrients may be present in one mixture, etc.

For industrial use, the connection means (300), the container (11), the first container (110) and the second container (210) are preferably made of stainless steel or copper.

In the framework of the invention, with porous stone is meant a porous solid substance that has the ability to diffuse gas into a liquid.

Examples of porous elements include but are not limited to air-stones (such as those used for fishtanks and aquariums), limewood and carborundum, preferably carborundum.

Preferably, the porous element(s) (13, 130 and/or 230) is/are coated with a non-sticking coating, for instance with a coating of polytetrafluoroethylene (also known as Teflon®).

The aqueous medium comprising water that is added in the process of the invention can be of any purity, but is preferably of a constant composition so that the algae oil production can be controlled. Preferably, the aqueous medium comprising water, for example consists of water and preferably the water used is demineralized and may also be sterilized (i.e. free of microorganisms that may contaminate the algae culture). Also, depending on the type of algae chosen, fresh water (for fresh water algae) or salt water, for example sea water (for marine algae) may be used. The demineralized water may be obtained from any source, but for efficiency reasons, the condensation water that is produced when flue/off-gases that contain CO₂ are cooled before CO₂ is captured may be used.

The aqueous medium comprising water that is added in the process of the invention may comprise biocides that inhibit the growth of microorganisms other than the algae, e.g. benzisothiozoninone or methylsothiazoninone or alternatively, the biocides may be added as a separate component or may be add as a mixture with one or more of the other components to be added in the process of the invention (CO₂, nutrients, surfactant, aqueous medium comprising water, algae).

Nutrients, together with CO₂ and light provide the material for algae to grow. Therefore, nutrients are added in the process of the invention. The skilled person is aware of the different type of chemical salts (nutrients) used in algae growth media.

Typically, algae growth media comprise the following constituents, water, nitrogen, phosphorus, vitamin B12, iron chloride, copper sulfate, silicate and sodium EDTA.

Recipes of various algae growth media and kits are available to the skilled person from for example the University of Texas at Austin, the University of Cologne, ACOI (Portugal), CCMP or Bigelow Laboratory for Ocean Sciences or from Eko Gea (Slovenia).

Since nutrients for marine algae are present in seawater, nutrients may also be added in the form of seawater. In this case, the aqueous medium may already comprise water and nutrients, for example the aqueous medium may comprise seawater. In case seawater is used, it is possible but not necessary to add nutrients as a separate component.

The CO₂ that is converted into algae oil may come from any source, but is preferably captured from industrial off- (or flue) gases, more preferably from off- (or flue) gases from power plants, for example from power plants that burn coals to generate electricity.

CO₂ may first be captured from industrial off- (or flue) gases using methods known in the art, for example CO₂ may be captured by using solid structures, for example natural stone (e.g. as described in EP1495794A1), carbon black or by amine compounds (e.g. the amine compounds as described in US6547854B1). The CO₂ captured in the solid structure may be then washed out of the solid structure using an aqueous medium comprising water to form an aqueous medium comprising water and CO₂. Tensides may be added to the aqueous medium comprising water used to wash the CO₂ out of the solid structures to increase the solubility of CO₂ in water.

Therefore, the second liquid may be an aqueous medium comprising water, CO₂ and also other ingredients, such as tensides. Alternatively, tensides may be added as a separate component or may be add as a mixture with one or more of the other components to be added in the process of the invention (CO₂, nutrients, surfactant, aqueous medium comprising water, algae).

Alternatively, CO₂ may be added as a gas for mixing with the other components according to the process of the invention.

Tensides which may be used to increase the solubility of CO₂ in water are known to the skilled person and include anionic tensides, such as metal salt based anionic tensides, but also e.g. the triethylamine salt of a secondary alkane sulfonate.

The surfactant is preferably chosen from the group of anionic surfactants, non-ionic surfactants and mixtures thereof. Either a single surfactant or a mixture of surfactants may be used, preferably a mixture of an anionic and a non-ionic surfactant is used. Representative surfactants include: sodium lauryl sulfate (SLS) and other alkyl sulfates, such as sodium dodecyl sulfate (SDS), sodium lauryl ether sulfate (SLES) and ammonium lauryl sulfate; sodium dodecyl benzene sulfonate and other alkyl and aryl sulfonates; sodium stearate and other fatty acid salts, alkylpolyethers and alkylpolyether sulfates; and polyvinyl alcohol and other non-ionic or polymeric surfactants, for example N-lauroylsarcosine sodium salt, cetyltrimethylammoniumbromide (CTAB), lauryldimethylamine-oxide (LDAO), sodium cholate, sodium deoxycholate and bis(2-ethylhexyl)sulfosuccinate sodium salt.

Examples of anionic surfactants include but are not limited to anionic surfactants based on permanent anions, for example sulfate, sulfonate or phosphate anions; or pH-dependent anions, for example carboxylate anions. Examples of anionic surfactants based on sulfates include but are not limited to alkyl sulfates, for example ammonium lauryl sulfate, sodium lauryl sulfate (also known as sodium dodecyl sulfate or SDS); alkyl ether sulfates, for example sodium laureth sulfate (also known as sodium lauryl ether sulfate or SLES) or sodium myreth sulfate. Examples of anionic surfactants based on sulfonates include but are not limited to docusates, for example dioctyl sodium sulfosuccinate. Examples of anionic surfactants based on phosphates include but are not limited to alkyl aryl ether phosphates and alkyl ether phosphate. Examples of anionic surfactants based on carboxylates include but are not limited to alkyl carboxylates, fatty acid salts, for example sodium stearate or sodium lauroyl sarcosinate. Preferably as anionic surfactant, sodium lauryl sulfate (also known as sodium dodecyl sulfate or SDS) or sodium laureth sulfate (also known as sodium lauryl ether sulfate or SLES) are used.

Examples of nonionic surfactants include but are not limited to fatty alcohols, for example cetyl alcohol, stearyl alcohol, cetostearyl alcohol or oleyl alcohol; polyoxyethylene glycol alkyl ethers of general formula CH₃-(CH₂)₁₀₋₁₆-(O-C₂H₄)₁₋₂₅-OH, for example octaethylene glycol monododecyl ether or pentaethylene glycol monododecyl ether; polyoxypropylene glycol alkyl ethers of general formula CH₃-(CH₂)₁₀₋₁₆-(O-C₃H₆)₁₋₂₅-OH, glucoside alkyl ethers of general formula CH₃-(CH₂)₁₀₋₁₆-(O-Glucoside)₁₋₃-OH, for example decyl glucoside, lauryl glucoside or octyl glucoside; polyoxyethylene glycol octylphenol ethers of general formula C₈H₁₇-(C₆H₄)-(O-C₂H₄)₁₋₂₅-OH, for example Triton X-100, polyoxyethylene glycol alkylphenol ethers of general formula C₉H₁₉-(C₆H₄)-(O-C₂H₄)₁₋₂₅-OH, for example nonoxynol-9; glycerol alkyl ester, for example glyceryl laurate; polyoxyethylene glycol sorbitan alkyl esters, for example polysorbates; sorbitan alkyl esters; cocamide MEA; cocamide DEA; dodecyldimethylamine oxide; and block copolymers of polyethylene glycol and polypropylene glycol, for example poloxamers. Preferably, as nonionic surfactant cocamide MEA or cocamide DEA is used.

For example, the non-ionic surfactant may be chosen from the group of cetyl alcohol, stearyl alcohol, cocamide MEA and cocamide DEA and/or the anionic surfactant may be chosen from the group of sodium dodecyl sulfate, sodium lauryl ether sulfate, sodium lauryl sulfate and ammonium lauryl sulfate.

The amount of surfactant used may for example be at least 0.1 wt%, for example at least 0.15wt%, for example at least 2wt%, for example at least 0.3 wt% based on water and /or at most 0.6wt%, for example at most 0.5wt%, for example at most 0.4wt% based on water. Preferably, in case an anionic surfactant is used, the amount of anionic surfactant used is at least 0.1 wt%, for example at least 0.15wt% and/or at most 0.5, for example at most 0.45wt% based on water.

Preferably, in case a non-ionic surfactant is used, the amount of non-ionic surfactant is at most 0.15wt% and/or for example at least 0wt%, for example at least 0.05wt% based on water.

In case the surfactant is chosen from a mixture of an anionic surfactant and a non-ionic surfactant, the mixture may be prepared for example by mixing water with 5-15wt% anionic surfactant and at most 5wt% non-ionic surfactant to make a 'surfactant concentrate', which concentrate may then be added to the process of the invention for example in an amount of 3 to 5wt% based on water added in the process of the invention.

In the process of the invention, in step b) the algae-containing bubbles are exposed to light causing the algae to produce algae oil. The light to which the algae-containing bubbles are exposed may be any light that can be used by the algae chosen for the production of algae oil. Certain frequencies of light may cause a higher production of algae oil than others and the light frequency may be adapted such that the highest production of algae oil is obtained.

The light used in the process of the invention may be artificial light, but preferably, the light used is ambient light, preferably daylight as daylight is a cheap and abundant source of light. The frequency of the light to which the algae-containing bubbles are exposed can also be adapted if daylight is used, by using specific filters or coatings on the reactor in which the algae-containing bubbles are present to filter through specific light frequencies only.

The algae-containing bubbles are exposed to light. This exposure may be done while the algae-containing bubbles are in a unit wherein the light can be transmitted, for example a transparent mixing unit as described above, or an open pond reactor may be used. Preferably, for reasons as explained above, algae oil is produced in a photobioreactor, which is a closed system.

Therefore, in the process of the invention, the algae-containing bubbles are preferably transferred into a photobioreactor, more preferably into a tubular photoreactor through which the algae-containing bubbles are transported in a continuous mode. This has the advantage that after transport of the algae-containing bubbles through the length of the tube, the composition of the algae-containing bubbles will be substantially uniform. In this way, a controlled production process for the production of algae oil is obtained. This as opposed to a batch process, wherein it is more difficult to predict the composition of the algae-containing bubbles after the elapse of time.

For purpose of the invention, photobioreactor is defined as a transparent closed system in which there is no direct exchange of gases and contaminants with the environment comprising an inlet and an outlet for the dispersions of the algae-containing bubbles respectively the algae oil containing bubbles.

There are many types of photobioreactors available and known to the skilled person. Designs of photobioreactors aim at 1) having a maximal volume of parts of the photobioreactor where the light is transmitted and 2) providing a uniform exposure of the algae culture to light and a uniform illumination of the culture surface in order to attain high productivity.

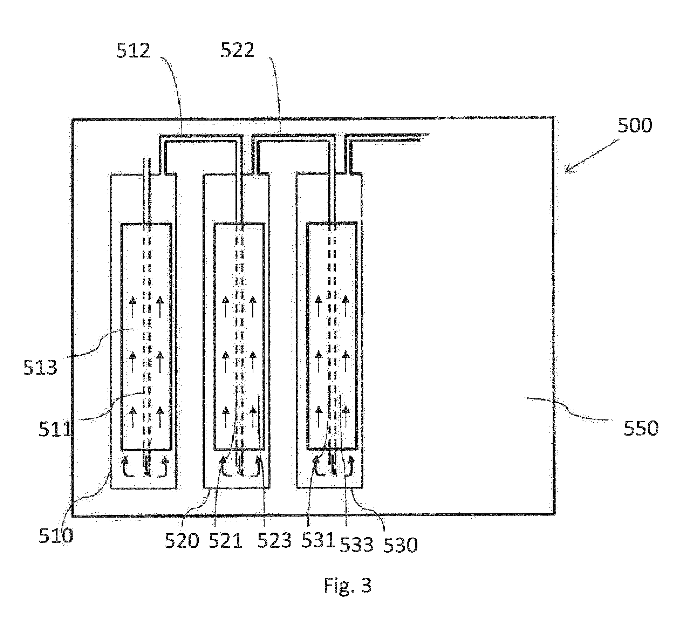

The photobioreactor as described in Figure 3 provides all this;

Figure 3 (Fig. 3) schematically illustrates a side view of an embodiment of the photobioreactor 500 according to the present invention. The photobioreactor in this embodiment comprises a substantially transparent first reaction unit 510, a substantially transparent second reaction unit 520 and a substantially transparent third reaction unit 530. The first reaction unit 510 and the reaction unit 520 are connected by a first connection means 512. Similarly, the second reaction unit 520 and the third reaction unit 530 are connected by a second connection means 522. The first and the second connection means 512 and 522 are hollow and may e.g. be tube-shaped. The reaction units 510, 520, 530 of the photobioreactor may be placed on a light reflecting sheet 550, for example a sheet of stainless steel, to increase the efficiency of the transmission of light and heat into the reaction units.

The photobioreactor 500 may be placed horizontally, e.g. on a flat roof or vertically, e.g. against a facade of a house of building or at an angle. Since in industry, many storage facilities and industrial (factory) buildings have flat roofs, it is especially desirable to place the photbioreactor 500 horizontally. An advantage of the photobioreactor 500 is that it is not critical for efficient production of algae oil at which angle it is placed to the light.

As is apparent to the skilled person, the photobioreactor 500 may comprise further substantially transparent reaction units if it is desired to increase the length of the photobioreactor, for example to increase the time of exposure of the algae-containing bubbles to the light.

The first reaction unit 510 is filled with a liquid medium, e.g. water. The first reaction unit 510 may comprise a transport channel 511 inserted in the liquid medium. A suspension of algae-containing bubbles can be led through the transport channel 511 into the reaction unit 510. The transport channel 511 is hollow and may e.g. be tube-shaped. The flow of the suspension of algae-containing bubbles may be effected by any means known, for transporting liquids, for example by one or more pumps (not shown).

As schematically represented in figure 3, each of the reaction units 510, 520, 530 may further comprise therein a respective light reflection means 513, 523, 533, which may for example be in the form of a flat surface extending over part of the respective transport channel 511, 521, 531 or for example the light reflection means 513, 523, 533 may also be curved, e.g. like a part of a cylinder.. The light reflection means 513, 523, 533 may for example be a sheet of tinoxide, which may be coated with a self-cleaning coating to minimize fouling.

The light reflection means 513, 523, 533 may be placed in parallel to the light reflecting plate 550, but the angle between the light reflection means 513, 523, 533 and the light reflecting plate 550 may be adjusted for optimal illumination. The light reflection means 513, 523, 533 is optional and is preferably present in a photobioreactor in milder climates. In these milder climates, the heat that is also reflected by the light reflection means is used for the culturing of the algae. In more tropical climates, there is no need for additional heating of the algae culture and there the light reflection means is preferably not present in the photobioreactor..

The algae in the algae-containing bubbles receive direct or reflected light and are allowed to produce algae oil by photosynthesis while the suspension flows through the reaction unit 510 and the transport channel (if the transport channel is also substantially transparent) 511 and the further reaction units (e.g. 520, 530, etc) and transport channels.

The suspension of the algae-containing bubbles can be transported from the first reaction unit 510 to the second reaction unit 520 via the connection means 511. The connection means 512 is connected to a transport channel 521. It is noted that the connection means 512 and the transport channel 521 may be one element or two different elements.

In a similar manner as described with respect to the first reaction unit 510, the algae in the algae-containing bubbles are allowed to produce algae oil by photosynthesis while the suspension flows through the transport channel (if the transport channel is also substantially transparent) 521 and the second reaction unit 520. The suspension flows through the second connection means 522 to the third reaction unit 530 wherein algae oil is produced in a similar way. The connection means 511,512 may also be substantially transparent.

The substantially transparent reaction units may be made of any substantially transparent material, for example they may be made of acrylics, glass or polycarbonate. A special type of transparent glass may be obtained from NARVA Kft. The reaction units may be coated. For example, the reaction units may be coated with a material that allows selective transmission of certain light frequencies, which may increase the production of algae oil. Also, the reaction units may be coated with a self-cleaning coating, e.g. a coating of nano particles of silicon dioxide that is sintered to the transparent material of the reaction units, to decrease the influence of weathering to the reaction units and to maintain a high efficiency of the transmission of light. Such self-cleaning coating may be present on both the outside and on the inside of the reaction units as well as on the optional light reflection means.

The transport channel may be made for example of a non-transparent material, for example copper. The transport channel is used to lengthen the time during which the algae are present in the photobioreactor.

In order to maintain/obtain/retain the temperature that is most optimal for the growth of the algae and for production of algae oil, the photobioreactor may be equipped with a heating or cooling system.

Preferably, the materials from which the photobioreactor is prepared are light-weight as they should not add too much weight to the building (on)to which the photobioreactor may be placed.

The photobioreactor may contain units to monitor the production of the algae oil, for example an optical cell density sensor may be placed to determined the density of the algae culture inside the algae-containing bubbles. If the density is low, the flow of the dispersion of algae-containing bubbles may be decreased to lengthen the time the dispersion is in the bioreactor. Alternatively and/or additionally, the dispersion of algae-containing bubbles may be circulated again and again through the photobioreactor until the algae-containing bubbles have become algae-oil containing bubbles that are ready for harvesting. Also, the photobioreactor may contain a light sensor, a conductivity sensor, a recirculation pump, a pump to harvest the algae-containing bubbles, a CO₂ sensor, a pH sensor, an oxygen meter etc.

The invention therefore, also relates to a process according to the invention, wherein in step b) the aqueous dispersion of algae-containing bubbles is transferred into a photobioreactor, preferably a tubular photobioreactor and subsequently exposed to light. and in particular to a process according to the invention, wherein step b) is performed in a photobioreactor (500) comprising a substantially transparent first reaction unit 510 and optionally substantially transparent further reaction units and optionally connecting means to connect the first reaction unit to the optional further reaction units
wherein step b) involves
- transferring the aqueous dispersion of algae-containing bubbles from the mixing unit to the photobioreactor
- transporting the algae-containing bubbles through the photobioreactor and
- exposing the aqueous dispersion of algae-containing bubbles to light to produce an aqueous dispersion of algae-oil containing bubbles.

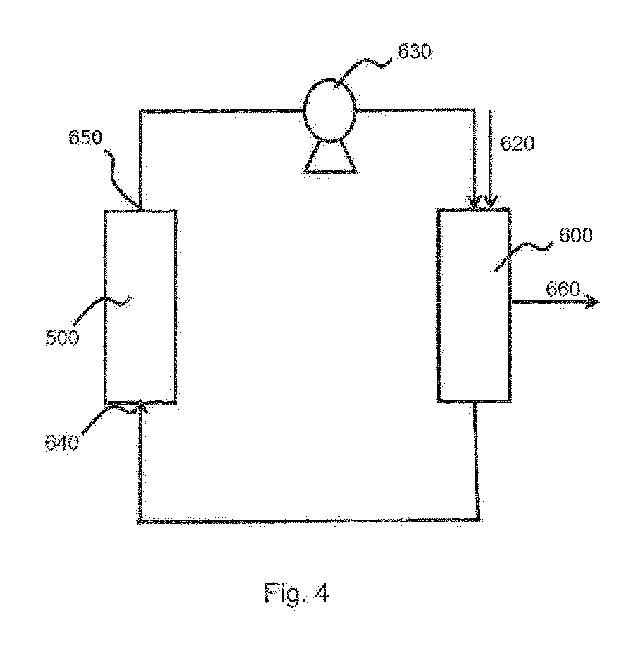

The dispersion of algae-containing bubbles or algae-oil containing bubbles may be recirculated through the photobioreactor. For example, the recirculation may be performed as schematically represented by Figure 4 (Fig. 4).

Figure 4 (Fig. 4) schematically illustrates the recirculation of the bubbles through the photobioreactor (500). The bubbles may be recirculated from the photobioreactor (500) to the outlet of the photobioreactor (650) using a recirculation pump (630) via a recirculation unit with an overflow separator (600) and back via the inlet of the photobioreactor (640) to the photobioreactor. The overflow (660) coming from the overflow separator may be further processed to obtain the algae-oil from the algae-oil containing bubbles.

In a further aspect, the invention relates to a process according to the invention further comprising the step of c) obtaining the algae oil from the algae-oil containing bubbles.

Obtaining the algae-oil from the algae-oil containing bubbles may be done in several ways.

Dewatering of the dispersion of algae-oil containing bubbles might be advantageous, since dewatering limits the volume that needs to be handled for obtaining the algae-oil. Dewatering can be done using methods known in the art, for example by using a centrifuge or a filter.

In any case, the algae-oil containing bubbles need to be broken before the algae-oil can be obtained. Breaking of the algae-oil containing bubbles may be done for example mechanically by using devices that produce shear forces, such as a press. Breaking of the algae-oil containing bubbles may also be done using an in-line mixer, such as an in-line mixer that is commercially available from Alfalaval. Preferably, the algae-oil containing bubbles are broken by using an ultrasonic cleaner (that is not submersible), preferably at a frequency in the range from 40 to 45kHz. Therefore, the invention relates to a process according to the invention, wherein step c) involves
- breaking the algae-oil containing bubbles using an ultrasonic cleaner at a frequency in the range from 40 - 45 kHz.

Furthermore, the algae-oil needs to be extracted from the algae. This can be done mechanically, for example using an expression/expeller press or ultrasonic-assisted extraction or chemically, for example by extraction using hexane, by Soxhlet extraction or by extraction using a supercritical fluid.

In case the algae-oil containing bubbles are broken by using an ultrasonic cleaner, the algae-oil is simultaneously extracted from the algae, which makes this a very elegant method of obtaining the algae-oil from the algae-oil containing bubbles.

Preferably, the algae are killed in the process of obtaining the algae-oil from the algae-oil containing bubbles. For example, the algae may be killed by exposing them to lethal amounts of UV-light. Details of such process are known to the person skilled in the art as it is a common procedure to kill algae or prevent the growth thereof.

The algae may be killed at any time during the process of obtaining the algae-oil from the algae-oil containing bubbles, for example the algae may be killed before or after the algae-oil containing bubbles are broken, but are preferably killed before the algae-oil is separated from the other components, more preferably before the algae-oil containing bubbles are broken.

The algae-oil obtained also needs to be separated from the other components present in the algae-oil containing bubbles.

The algae in the algae-oil containing bubbles will have produced three main components: carbohydrates, algae residue and lipids (the algae-oil). The carbohydrates may be converted into sugars using methods known in the art and subsequently converted into methanol and/or ethanol. The algae residue may for example be used as feed, as fertilizer or for combustion.

The algae-oil may be converted into biofuel, for example biodiesel by methods known in the art, such as for example transesterification.

The invention therefore also relates to a process for the preparation of biofuel comprising the process of the invention and the step of converting the algae oil into biofuel.

The ratio of the three main components that are produced depends amongst others on the algae and the conditions chosen. Therefore, the process of the invention can also be used for the production of any other algae-based product.

Separation of the algae-oil from the other components may be done using conventional separation techniques, for example the solid components may be separated from the liquid components using a filter and the liquid components may be separated from one another using conventional phase separation techniques, such as for example centrifugation.

Separation of these components may for example be done using an algae decanter/centrifuge separator as is commercially available from for example GEA Westfalia Separator Group GmbH.

Therefore, the invention also relates to a process according to the invention, wherein step c) involves
- killing the algae by exposing the algae to lethal amounts of UV-light
- breaking the bubbles using an ultrasonic cleaner at a frequency in the range from 40 - 45 kHz and
- separating the algae-oil from the other components to obtain the algae oil and in particular to a process according to the invention, wherein step c) involves
- dewatering of the dispersion of algae-oil containing bubbles
- killing the algae by exposing the algae to lethal amounts of UV-light
- breaking the bubbles using an ultrasonic cleaner and
- separating the algae oil from the other components to obtain the algae oil.

### Brief description of the figures

Figure 1 (Fig. 1) schematically illustrates a mixing unit 10.
Figure 2 (Fig. 2) schematically illustrates a mixing unit 100 and a second mixing unit 200.
Figure 3 (Fig. 3) schematically illustrates a side view of an embodiment of the photobioreactor 500 according to the present invention.
Figure 4 (Fig. 4) schematically illustrates the recirculation of the bubbles through the photobioreactor (500).

It is noted that the invention relates to all possible combination of features recited in the description, especially the claims.

The invention will now be elucidated by way of the following examples without however being limited thereto.

### Examples

### Photobioreactor

In the following examples, a photoreactor as exemplified by Figure 3 was used. As used in the experiments described below, the photobioreactor (500) consisted of 20 reaction units (510, 520, 530, etc.) made of cladding tubes of a length of 2 m, each of which was 58mm in diameter. The cladding tubes were obtained from NARVA Kft. The cladding tubes were made of highly transparent special glass, which was coated on both the outside and on the inside with nano particles of silicon dioxide after which the layers were sintered into the surfaces, making the glass smudge-proof. The cladding tubes were connected via copper connections means (512, 522, etc.). Light reflection means were present ine the reaction units in the form of tinoxide sheets. A stainless steel surface (550) was placed behind the photobioreactor (500). The photobioreactor was operated in vertical position and the whole photobioreactor measured 220 x 150 cm.

The reaction units of the photobioreactor were mounted in an inlet manifold and an outlet manifold which were equipped with an inlet respectively an outlet for the algae-containing respectively the algae-oil containing bubbles and further more with a light sensor, a conductivity sensor, two recirculation pumps, a CO₂ sensor, an O₂ sensor and a pH sensor, a temperature sensor and a heating and a cooling element. The bubbles were recirculated over a recirculation unit (600) as depicted in Figure 4 (Fig. 4) using the recirculation pump (630). The recirculation pump used was the Aqua EL350 (350l/h) pump from Aqua, Taiwan.

### Mixing units

In the following examples, two mixing units, as exemplified in Figure 2 were used. As used in the experiments described below, mixing unit (100) consisted of a 1 L plastic container wherein a polytetrafluoroethylene coated carborundum stone (130) was placed. A rod-shaped submersible ultrasonic transducer (140) from Hielscher Ultrasonics GmbH (UIP1000hd, 1000W) was placed in the container. Mixing unit (100) was connected to mixing unit (200) via a plastic tube (300). Mixing unit (200) consisted of a 1 L plastic container wherein a polytetrafluoroethylene coated carborundum stone (230) was placed. A rod-shaped submersible ultrasonic transducer (140) from Hielscher Ultrasonics GmbH (UIP1000hd, 1000W) was placed in the container.

### Process for the preparation of algae-oil

The mixing units were filled with demineralized water and the water was recirculated through through the carborundum stones and the mixing units for 5 minutes to remove air-bubbles present in the mixing units and in the carborundum stones. The pH, the optical density, the oxygen and CO₂ levels were measured to obtain reference values.

Fain tenside mixture WP3 as obtained from Oy Faintend Ltd in Finland was added to the demineralized water through the carborundum stone of the first mixing unit in an amount of 3-5 wt% based on the demineralized water and was recirculated through the carborundum stones and the reaction units till a stable pH of 5 - 7 was reached.

CO₂ from a gascilinder of 1000psi was continuously added at a pressure of 22psi through the carborundum stone into the first mixing unit.

Nutrients were added to the first mixing unit through the carborundum stone in the form of 2ml Eko-Gro liquid (from EkoGEA, Slovenia) per liter demineralized water.

Then, 3-5wt% based on the demineralized water of a surfactant mixture from Thurn Produktie GmbH, Much, Germany comprising 5-15wt% anionic surfactant (mainly sodiumdodecylsulfate and sodium lauryl ether sulfate), less than 5wt% of a non-ionic surfactant (cocamide DEA and cocamide MEA) and small amounts of the biocides benzisothiozolinone and methylsothiozoninone was added through the carborundum stone into the first mixing unit.

The submersible ultrasonic transducer in the first mixing unit (110) was then operated at a frequency of 20-25kHz and a milky white dispersion of bubbles was obtained. The overflow of bubbles flowed to the second mixing unit (210) via the plastic tube (300) and entered into the second mixing unit via the carborundum stone.

Then, 5wt% based on water of a *Parachlorella kessleri* inoculation culture was added to the second mixing unit through the carborundum stone.

The milky white dispersion of bubbles obtained from the first mixing unit and now present in the second mixing unit was mixed with the *Parachlorella kessleri* inoculation culture by operating the submersible ultrasonic transducer in the second mixing unit at a frequency of 20 to 25kHz. A milky white dispersion of algae-containing bubbles was obtained. The overflow of the dispersion of algae-containing bubbles was pumped to the photobioreactor.

The dispersion of algae-containing bubbles was recirculated with a continuous flow through the recirculation unit and back into the photobioreactor for 5 days (5 times 24 hours) at 36°C. The amount of daylight received by the algae-containing bubbles was 16 hours per 24 hours and it was dark for 8 hours per 24 hours. The flow of the dispersion of algae-containing bubbles was set to 4.2L/min.

When the optical density increased (the algae-containing bubbles turned from green to dark green) and the oxygen sensor showed an increase in oxygen (which confirmed that photosynthesis occurred), the (now) algae-oil containing bubbles were harvested as follows:
The dispersion of algae-oil containing bubbles was transported into a transparent container wherein the dispersion was illuminated with UV-light for 1 hour by using a Philips facial tanning machine (Philips Cleo, type UV HB175 with 4 times 15W UV tubelamps). Subsequently, the dispersion was pumped to a glass container of 20L, which container was filled with 10L of the dispersion and which container was equipped with a separation filter in the middle of the tank and the dispersion was pumped through the filter. The filter used was a 10mm thick fumehood filter which was a combination of a fat filter and a carbon filter (type EAN8711261000044, Bauknecht). The material that was retained by the filter was subsequently pumped into an ultrasonic cleaner (type Emmi20hc obtained from EMAG, Germany). This ultrasonic cleaner was a stainless steel container that contained an ultrasonic cleaner on the outside on the bottom. The ultrasonic cleaner was operated at a frequency of 40-45kHz until a homogeneous mixture was obtained.

The formed homogeneous mixture was subsequently passed through a MAN fuel filter (height 184mm, diameter 84mm, nr. 5112503-0059) for a dewatering step. The filter separated the algae oil-containing fraction from an aqueous fraction. The aqueous fraction was recirculated to the first mixing unit (110) and reused.

The algae oil-containing fraction was allowed to settle and after 24 hours a three-phase system was formed. The three-phase system contained an algae-oil fraction on top of approximately 30ml, a carbohydrate/protein fraction in the middle of approximately 60 ml and a rest phase of approximately 10 ml.

The oil fraction obtained may be further converted into biofuel as known per se. The carbohydrate/protein fraction may be converted into methanol and/or ethanol as known per se.

The example may be repeated with other algae, for example with the marine algae *Dunaliella saline,* or with *Dunaliella tertiolecta.* The conditions and amounts as described above may be used; except for the following: The pH is preferably in the range of 8-12, preferably in the range of 10-12; the temperature in the photobioreactor is preferably chosen in the range of 28-30°C. In case marine algae are used to prepare the algae-containing bubbles, seawater (for example seawater from the Indian or Pacific Ocean) instead of demineralized water may be used and since nutrients are already present in seawater, there is no need to separately add nutrients.

## Claims

1. Process for the production of algae oil comprising the steps of
a) mixing algae, nutrients, CO₂, a surfactant and an aqueous medium comprising water to form an aqueous dispersion of algae-containing bubbles comprising algae, nutrients, CO₂, and water and
b) exposing the dispersion to light to produce algae oil-containing bubbles comprising algae oil.

2. Process according to claim 1, wherein the surfactant is chosen from the group of anionic surfactants, non-ionic surfactants and mixtures thereof

3. Process according to claim 1 or claim 2, wherein step a) is performed by operating a submersible ultrasonic transducer (14; 140; 240), preferably at a frequency in the range from 20 to 25kHz, to form the algae-containing bubbles (15; 150; 250).

4. Process according to any one of claims 1-3, wherein step a) is performed using a mixing unit (10) comprising
- a container (11) containing a first liquid (12)
- a porous element (13) which is at least partially submerged in the first liquid and
- a submersible ultrasonic transducer (14) which is at least partially submerged in the first liquid and wherein the first liquid is an aqueous medium comprising water, wherein step a) involves
- leading a second liquid and/or a first gas stream into the container, preferably through the porous element, wherein the second liquid is an aqueous medium comprising water and CO₂, wherein the first gas stream is a gas comprising CO₂
- adding the nutrients, algae and the surfactant to the container, preferably through the porous element and
- operating the submersible ultrasonic transducer at a frequency in the range from 20 to 25kHz to produce the aqueous dispersion of algae-containing bubbles (15).

5. Process according to any one of claims 1-3, wherein step a) is performed using a mixing unit (100) comprising
- a first container (110) containing a third liquid (120),
- a porous element (130) which is at least partially submerged in the third liquid and
- a first submersible ultrasonic transducer (140) which is at least partially submerged in the third liquid and
wherein the third liquid is an aqueous medium comprising water and
a second mixing unit (200) comprising
- a second container (210) containing a fourth liquid (220),
- a porous element (230) which is at least partially submerged in the fourth liquid and
- a second submersible ultrasonic transducer (240) which is at least partially submerged in the fourth liquid and wherein the fourth liquid is an aqueous medium comprising water and
a transporting means (300) connecting the third liquid to the fourth liquid wherein step a) involves
- leading a second liquid and/or a second gas stream into the second container, preferably through the porous element, wherein the second liquid is an aqueous medium comprising water and CO₂, wherein the second gas stream is a gas comprising CO₂
- adding algae to the second container, preferably through the porous element
- operating the first submersible ultrasonic transducer at a frequency in the range from 20 to 25kHz to produce an aqueous dispersion of bubbles (150) comprising nutrients, CO₂ and water and
- subsequently leading the aqueous dispersion of bubbles comprising nutrients, CO₂, and water into the second container, preferably through the porous element using the transporting means and
- operating the second submersible transducer at a frequency in the range from 20 to 25kHz to produce an aqueous dispersion of algae-containing bubbles comprising algae, CO₂, nutrients and water (250).

6. Process according to any one of claims 1-5, wherein the light used in step b) is daylight.

7. Process according to any one of claims 1-6, wherein in step b) the aqueous dispersion of algae-containing bubbles is transferred into a photobioreactor, preferably a tubular photobioreactor and subsequently exposed to light.

8. Process according to any one of claims 1-7, wherein step b) is performed in a photobioreactor (500) comprising a substantially transparent first reaction unit 510 and optionally substantially transparent further reaction units and optionally connecting means to connect the first reaction unit to the optional further reaction units
wherein step b) involves
- transferring the aqueous dispersion of algae-containing bubbles from the mixing unit to the photobioreactor
- transporting the algae-containing bubbles through the photobioreactor and
- exposing the aqueous dispersion of algae-containing bubbles to light to produce an aqueous dispersion of algae-oil containing bubbles.

9. Process according to any one of claims 1-8 further comprising the step of c) obtaining the algae oil from the algae-oil containing bubbles.

10. Process according to claim 9, wherein step c) involves
- breaking the algae-containing bubbles using an ultrasonic cleaner at a frequency in the range from 40 - 45 kHz.

11. Process according to claim 9 or claim 10, wherein step c) involves
- killing the algae by exposing the algae to lethal amounts of UV-light
- breaking the bubbles using an ultrasonic cleaner, preferably at a frequency in the range from 40 - 45 kHz and
- separating the algae-oil from the other components to obtain the algae oil.

12. Process according to any one of claims 9-11, wherein step c) involves
- dewatering of the dispersion of algae-oil containing bubbles
- killing the algae by exposing the algae to lethal amounts of UV-light
- breaking the bubbles using an ultrasonic cleaner and
- separating the algae oil from the other components to obtain the algae oil.

13. Process for the preparation of biofuel comprising the process of any one of claims 1-12 and the step of converting the algae oil into biofuel.

14. Process according to any one of claims 1-13, wherein the algae are chosen from the group of *Parachlorella kessleri, Chlorella vulgaris, Emilyania Huxleyi coccolith, Dunaliella tertiolecta* and *Dunaliella saline.*

15. Process according to any one of claims 2-14, wherein the non-ionic surfactant is chosen from the group of cetyl alcohol, stearyl alcohol, cocamide MEA and cocamide DEA and/or wherein the anionic surfactant is chosen from the group of sodium dodecyl sulfate, sodium lauryl ether sulfate, sodium lauryl sulfate and ammonium lauryl sulfate.

## Patentansprüche

1. Verfahren zur Herstellung von Algenöl, das die folgenden Schritte umfasst
a) Mischen von Algen, Nährstoffen, CO₂, einem Tensid und einem wässrigen Medium, das Wasser umfasst, zur Bildung einer wässrigen Dispersion von Algen enthaltenden Blasen, die Algen, Nährstoffe, CO₂ und Wasser umfassen und
b) Aussetzen der Dispersion gegenüber Licht zur Bildung von Algenöl enthaltenden Blasen, die Algenöl umfassen.

2. Verfahren nach Anspruch 1, wobei das Tensid ausgewählt wird aus der Gruppe von anlonischen Tenslden, nicht Ionischen Tensiden und Gemischen davon.

3. Verfahren nach Anspruch 1 oder Anspruch 2, wobei Schritt a) ausgeführt wird durch Betreiben eines tauchfähigen Ultraschallwandlers (14; 140; 240), vorzugsweise bei einer Frequenz im Bereich von 20 bis 25 kHz zur Bildung der Algen enthaltenden Blasen (15; 150; 250).

4. Verfahren nach einem der Ansprüche 1-3, wobei Schritt a) ausgeführt wird unter Verwendung einer Mischeinheit (10), die Folgendes umfasst:
- einen Behälter (11), der eine erste Flüssigkeit (12) enthält
- ein poröses Element (13), das wenigstens teilweise in die erste Flüssigkeit eingetaucht wird und
- einen tauchfählgen Ultraschallwandler (14), der wenigstens teilweise in die erste Flüssigkeit eingetaucht wird und wobei die erste Flüssigkeit ein wässriges Medium ist, das Wasser umfasst,
wobei Schritt a) Folgendes umfasst
- Einleiten einer zweiten Flüssigkeit und/oder eines ersten Gasstroms In den Behälter, vorzugsweise durch das poröse Element, wobei die zweite Flüssigkeit ein wässriges Medium ist, das Wasser und CO₂ umfasst, wobei der erste Gasstrom ein Gas ist, das CO₂ umfasst
- Hinzufügen der Nährstoffe, Algen und des Tensids zu dem Behälter, vorzugsweise durch das poröse Element und
- Betreiben des tauchfähigen Ultraschallwandlers bei einer Frequenz im Bereich von 20 bis 25 kHz zur Bildung der wässrigen Dispersion von Algen enthaltenden Blasen (15).

5. Verfahren nach einem der Ansprüche 1-3, wobei Schritt a) unter Verwendung einer Mischeinheit (100) ausgeführt wird, die Folgendes umfasst:
- einen ersten Behälter (110), der eine dritte Flüssigkeit (120) enthält,
- ein poröses Element (130), das wenigstens teilweise In die dritte Flüssigkeit eingetaucht wird und
- einen ersten tauchfähigen Ultraschallwandler (140), der wenigstens teilweise in die dritte Flüssigkeit eingetaucht wird und
wobei die dritte Flüssigkeit ein wässriges Medium Ist, das Wasser umfasst, und
eine zweite Mischeinheit (200), die Folgendes umfasst:
- einen zweiten Behälter (210), der eine vierte Flüssigkeit (220) enthalt
- ein poröses Element (230), das wenigstens teilweise in die vierte Flüssigkeit eingetaucht wird und
- einen zweiten tauchfähigen Ultraschallwandler (240), der wenigstens teilweise in die vierte Flüssigkeit eingetaucht wird und wobei die vierte Flüssigkeit ein wässriges Medium Ist, das Wasser umfasst, und
eine Transportvorrichtung (300), welche die dritte Flüssigkeit mit der vierten Flüssigkeit verbindet
wobei Schritt a) Folgendes umfasst:
- Einleiten einer zweiten Flüssigkeit und/oder eines zweiten Gasstroms in den zweiten Behälter, vorzugsweise durch das poröse Element, wobei die zweite Flüssigkeit ein wässriges Medium ist, das Wasser und CO₂ umfasst, wobei der zweite Gasstrom ein Gas ist, das CO₂ umfasst
- Hinzufügen von Algen zu dem zweiten Behälter, vorzugsweise durch das poröse Element
- Betreiben des ersten tauchfähigen Ultraschallwandlers bei einer Frequenz im Bereich von 20 bis 25 kHz zur Bildung einer wässrigen Dispersion von Blasen (150), die Nährstoffe, CO₂ und Wasser umfassen und
- daraufhin Einleiten der wässrigen Dispersion von Blasen, die Nährstoffe, CO₂ und Wasser umfassen in den zweiten Behälter, vorzugsweise durch das poröse Element unter Verwendung der Transportvorrichtung
- Betreiben des zweiten tauchfähigen Ultraschallwandlers bei einer Frequenz im Bereich von 20 bis 25 kHz zur Bildung einer wässrigen Dispersion von Algenenthaltenden Blasen, die Algen, CO₂, Nährstoffe und Wasser (250) umfassen.

6. Verfahren nach einem der Ansprüche 1-5, wobei das in Schritt b) verwendete Licht Tageslicht ist

7. Verfahren nach einem der Ansprüche 1-6, wobei In Schritt b) die wässrige Dispersion von Algen enthaltenden Blasen in einen Photobioreaktor überführt wird, vorzugsweise einen tubulären Photobioreaktor und daraufhin Licht ausgesetzt wird.

8. Verfahren nach einem der Ansprüche 1-7, wobei Schritt b) in einem Photobioreaktor (500) durchgeführt wird, der eine im Wesentlichen transparente erste Reaktlonseinheit (510) umfasst und wahlweise Im Wesentlichen transparente weitere Reaktionseinheiten und wahlweise Verbindungsvorrichtungen, um die erste Reaktionseinheit mit den wahlweisen weiteren Reaktionseinheiten zu verbinden
wobei Schritt b) Folgendes umfasst:
- Überführen der wässrigen Dispersion von Algen enthaltenden Blasen aus der Mischeinheit in den Photobioreaktor
- Transportieren der Algen enthaltenden Blasen durch den Photobioreaktor und
- Aussetzen der wässrigen Dispersion Algen enthaltender Blasen gegenüber Licht zur Herstellung einer wässrigen Dispersion von Algenöl enthaltenden Blasen.

9. Verfahren nach einem der Ansprüche 1-8, das ferner den Schritt c) des Gewinnens des Algenöls aus den Algenöl enthaltenden Blasen umfasst.

10. Verfahren nach Anspruch 9, wobei Schritt c) Folgendes umfasst:
- Aufbrechen der Algenöl enthaltenden Blasen unter Verwendung eines Ultraschallreinigungsgeräts bei einer Frequenz im Bereich von 40-45 kHz.

11. Verfahren nach Anspruch 9 oder Anspruch 10, wobei Schritt c) Folgendes umfasst:
- Töten der Algen durch Aussetzen der Algen gegenüber tödlichen Mengen von UV-Licht
- Aufbrechen der Blasen unter Verwendung eines Ultraschallreinigungsgeräts, vorzugsweise bei einer Frequenz im Bereich von 40-45 kHz und
- Trennen des Algenöls von den anderen Bestandteilen, um das Algenöl zu erhalten.

12. Verfahren nach einem der Ansprüche 9-11, wobei Schritt c) Folgendes umfasst:
- Entwässern der Dispersion Algenöl enthaltender Blasen
- Töten der Algen durch Aussetzen der Algen gegenüber tödlichen Mengen von UV-Licht
- Aufbrechen der Blasen unter Verwendung eines Ultraschallreinigungsgeräts und
- Trennen des Algenöls von den anderen Bestandteilen, um das Algenöl zu erhalten.

13. Verfahren zur Herstellung von Blotreibstoff, welches das Verfahren nach einem der Ansprüche 1-12 umfasst sowie den Schritt des Umwandelns des Algenöls in Biokraftstoff.

14. Verfahren nach einem der Ansprüche 1-13, wobei die Algen ausgewählt sind aus der Gruppe von *Parachlorella kessleri, Chloreile vulgaris, Emilyanla Huxleyi coccolith, Dunaliella tertiolecta* und *Dunaliella saline.*

15. Verfahren nach einem der Ansprüche 2-14, wobei das nicht ionische Tensid ausgewählt wird aus der Gruppe von Cetylalkohol, Stearylalkohol, Cocoamid MEA und Cocoamid DEA und/oder wobei das anionische Tensid ausgewählt wird aus der Gruppe von Natriumdodecylsulfat, Natriumlaurylethersulfat, Natriumlaurylsulfat und Ammoniumlaurylsulfat.

## Revendications

1. Processus destiné à la production d'huile d'algues, comprenant les étapes de :
a) mélange des algues, des nutriments, du CO₂, d'un tensioactif et d'un milieu aqueux comprenant de l'eau afin de former une dispersion aqueuse de bulles contenant des algues, comprenant des algues, des nutriments, du CO2 et de l'eau, et
b) exposition de la dispersion à la lumière afin de produire des bulles contenant de l'huile d'algues, comprenant de l'huile d'algues.

2. Processus selon la revendication 1, dans lequel le tensioactif est choisi dans le groupe comprenant les tensioactifs anioniques, les tensioactifs non-ioniques et des mélanges de ceux-ci.

3. Processus selon la revendication 1 ou 2, dans lequel l'étape a) est effectuée grâce à l'utilisation d'un transducteur ultrasonique submersible (14 ; 140 ; 240), de préférence à une fréquence comprise entre 20 et 25 kHz, pour former les bulles contenant des algues (15 ; 150 ; 250).

4. Processus selon l'une quelconque des revendications 1 à 3, dans lequel rétape a) est effectuée à l'aide d'une unité de mélange (10) comprenant :
- un conteneur (11) contenant un premier liquide (12),
- un élément poreux (13) au moins partiellement immergé dans le premier liquide et
- un transducteur ultrasonique submersible (14) au moins partiellement immergé dans le premier liquide et où le premier liquide est un milieu aqueux comprenant de l'eau,
dans lequel l'étape a) inclut :
- le passage d'un second liquide et/ou d'un premier flux de gaz dans le conteneur, de préférence à travers l'élément poreux, où le second liquide est un milieu aqueux comprenant de l'eau et du CO₂, où le premier flux de gaz est un gaz comprenant du CO₂,
- l'ajout des nutriments, des algues et du tensioactif dans le conteneur, de préférence à travers l'élément poreux, et
- l'utilisation du transducteur ultrasonique submersible à une fréquence comprise entre 20 et 25 kHz afin de produire la dispersion aqueuse de bulles contenant des algues (15).

5. Processus selon l'une quelconque des revendications 1 à 3, dans lequel l'étape a) est effectuée à l'aide d'une unité de mélange (100) comprenant :
- un premier conteneur (110) contenant un troisième liquide (120),
- un élément poreux (130) au moins partiellement immergé dans le troisième liquide et
- un premier transducteur ultrasonique submersible (140) au moins partiellement immergé dans le troisième liquide et
où le troisième liquide est un milieu aqueux comprenant de l'eau et une deuxième unité de mélange (200) comprenant :
- un second conteneur (210) contenant un quatrième liquide (220),
- un élément poreux (230) au moins partiellement immergé dans le quatrième liquide et
- un second transducteur ultrasonique submersible (240) au moins partiellement immergé dans le quatrième liquide et où le quatrième liquide est un milieu aqueux comprenant de l'eau et
un moyen de transport (300) reliant le troisième liquide au quatrième liquide où l'étape a) inclut:
- le passage d'un second liquide et/ou d'un second flux de gaz dans le second conteneur, de préférence à travers l'élément poreux, dans lequel le second liquide est un milieu aqueux comprenant de l'eau et du CO₂, où le second flux de gaz est un gaz comprenant du CO₂,
- l'ajout d'algues au second conteneur, de préférence à travers l'élément poreux,
- l'utilisation du premier transducteur ultrasonique submersible à une fréquence comprise entre 20 et 25 kHz afin de produire une dispersion aqueuse de bulles (150) comprenant des nutriments, du CO₂ et de l'eau, et
- le passage ultérieur de la dispersion aqueuse de bulles comprenant des nutriments, du CO₂ et de l'eau dans le second conteneur, de préférence à travers l'élément poreux, grâce à l'utilisation du moyen de transport, et
- l'utilisation du second transducteur ultrasonique submersible à une fréquence comprise entre 20 et 25 kHz afin de produire une dispersion aqueuse de bulles contenant des algues, comprenant des algues, du CO₂, des nutriments et de l'eau (250).

6. Processus selon l'une quelconque des revendications 1 à 5, dans lequel la lumière utilisée dans l'étape b) est la lumière naturelle.

7. Processus selon l'une quelconque des revendications 1 à 6, dans lequel dans l'étape b) la dispersion aqueuse de bulles contenant des algues est transférée dans un photobioréacteur, de préférence un photobioréacteur tumulaire, puis exposée à la lumière.

8. Processus selon l'une quelconque des revendications 1 à 7, dans lequel l'étape b) est effectuée au sein d'un photobioréacteur (500), comprenant une première unité de réaction nettement transparente (510), ainsi que, facultativement, d'autres unités de réactions nettement transparentes, et facultativement, des moyens de connexion entre la première unité de réaction et les autres unités de réaction facultatives où l'étape b) inclut :
- le transfert de la dispersion aqueuse de bulles contenant des algues de l'unité de mélange au photobioréacteur,
- le transport des bulles contenant des algues à travers le photobioréacteur et
- l'exposition de la dispersion aqueuse contenant des bulles à la lumière afin de produire une dispersion aqueuse de bulles contenant de l'huile d'algues.

9. Processus selon l'une quelconque des revendications 1 à 8, comprenant en outre l'étape c), permettant d'obtenir l'huile d'algues à partir des bulles contenant l'huile d'algues.

10. Processus selon la revendication 9, dans lequel l'étape c) inclut
- l'éclatement des bulles contenant des algues à l'aide d'un nettoyeur à ultrasons à une fréquence comprise entre 40 et 45 kHz.

11. Processus selon la revendication 9 ou 10, dans lequel l'étape c) inclut
- la destruction des algues en les exposant à des quantités létales de lumière UV,
- l'éclatement des bulles à l'aide d'un nettoyeur à ultrasons, de préférence à une fréquence comprise entre 40 et 45 kHz et
- la séparation de l'huile d'algues des autres composants, afin d'obtenir l'huile d'algues.

12. Processus selon l'une quelconque des revendications 9 à 11, dans lequel l'étape c) inclut :
- l'extraction de l'eau de la dispersion de bulles contenant de l'huile d'algues,
- la destruction des algues en les exposant à des quantités létales de lumière UV,
- l'éclatement des bulles à l'aide d'un nettoyeur à ultrasons et
- la séparation de l'huile d'algues des autres composants, afin d'obtenir l'huile d'algues.

13. Processus destiné à la préparation de biocarburant, comprenant le processus selon l'une quelconque des revendications 1 à 12 et l'étape de conversion de l'huile d'algues en biocarburant.

14. Processus selon l'une quelconque des revendications 1 à 13, dans lequel les algues choisies font partie du groupe comprenant *Parachlorella kessleri, Chlorella vulgaris,* les coccolithes d*'Emiliania Huxleyi, Dunaliella tertiolecta* et *Dunaliella saline.*

15. Processus selon l'une quelconque des revendications 2 à 14, dans lequel le tensioactif non-ionique est choisi dans le groupe comprenant l'alcool cétylique, l'alcool stéarylique, la cocamide MEA et la cocamide DEA et/ou dans lequel le tensioactif anionique est choisi dans le groupe comprenant le dodécylsulfate de sodium, le lauryl éther sulfate de sodium, le laurylsulfate de sodium et le laurylsulfate d'ammonium.
